(19) Europäisches Patentamt
European Patent Office
Office européen des brevets

(11) Veröffentlichungsnummer: **0 093 363**
**B1**

(12) # EUROPÄISCHE PATENTSCHRIFT

(45) Veröffentlichungstag der Patentschrift: **14.01.87**

(21) Anmeldenummer: **83104006.8**

(22) Anmeldetag: **23.04.83**

(51) Int. Cl.[4]: **C 07 B 45/00**, C 09 B 49/00 // C07C149/34, C07C149/32, C09B49/12, C07C149/42, C07D333/34, C07D213/70, C07C149/41, C08G75/14, C09B49/06

(54) **Verfahren zur Herstellung von Disulfiden.**

(30) Priorität: **30.04.82 DE 3216126**

(43) Veröffentlichungstag der Anmeldung: **09.11.83 Patentblatt 83/45**

(45) Bekanntmachung des Hinweises auf die Patenterteilung: **14.01.87 Patentblatt 87/3**

(84) Benannte Vertragsstaaten: **CH DE FR GB IT LI**

(56) Entgegenhaltungen: **GB-A-582 638** **US-A-2 571 740**

(73) Patentinhaber: **CASSELLA Aktiengesellschaft, Hanauer Landstrasse 526, D-6000 Frankfurt am Main 61 (DE)**

(72) Erfinder: **Bauer, Wolfgang, Dr., Masurenstrasse 6, D-6457 Maintal 3 (DE)**
Erfinder: **Kühlein, Klaus, Dr., Fasanenstrasse 41, D-6233 Kelkheim/Taunus (DE)**

(74) Vertreter: **Urbach, Hans- Georg, Dr., Hanauer Landstrasse 526, D-6000 Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1987

**Beschreibung**

Die vorliegende Erfindung betrifft ein wirtschaftliches und ökologisch vorteilhaftes Verfahren zur Herstellung von aromatischen, heteroaromatischen oder Farbstoff-Disulfiden, mit Ausnahme von Nitrogruppen enthaltenden aromatischen, heteroaromatischen und Farbstoff-disulfiden, durch Reduktion der entsprechenden Sulfochloride mit Iodwasserstoffsäure oder mit Iodwasserstoffsäure liefernden Substanzen in Gegenwart von Reduktionsmitteln, die Iod zur Iodwasserstoffsäure überführen.

Aromatische Sulfonsäurechloride, beispielsweise Nitrobenzolsulfonsäurechloride oder Nitronaphthalinsulfonsäurechloride, werden mit Iodwasserstoffsäure ohne Verwendung eines organischen Lösungsmittels oder in Essigsäure, Ether oder Aceton zu den entsprechenden Dinitrodiaryl-disulfiden reduziert (vgl.z. B. Organic Synthesis 40, 80; Ber. dtsch. chem. Ges. 20, 1534 (1887); 21, 1099 (1888); 23, 958 (1890); 25, 2485 (1892); J. Chem. Soc. 1949, 3434). Die Reduktion von 2-Thienylsulfo-chlorid mit Iodwasserstoffsäure in Eisessig liefert 2,2'-Dithienyl-disulfid (J. Chem. Soc. 1948, 769). Diese Verfahren benötigen zur Reduktion von 1 mol Sulfochlorid gemäß der Reaktionsgleichung:

$2\ RSO_2Cl + 10\ HI \rightarrow R\text{-}S\text{-}S\text{-}R + 2\ HCl + 4\ H_2O + 5\ I_2$

5 mol Iodwasserstoffsäure und sind daher durch die hohen Kosten für Iodwasserstoff unwirtschaftlich.

Bei der Reduktion von Nitrobenzolsulfochloriden mit Phosphor in Gegenwart von Iodwasserstoffsäure bzw. Iod in verdünnter Essigsäure werden ebenfalls Dinitrodiphenyl-disulfide erhalten (DL 184 985 (1970)). p-Toluolsulfochlorid wird mit Phosphor und Kaliumiodid in wäßrigem Reaktionsmedium in 4,4'-Dimethyldiphenyldisulfid überführt (Jap. Patentschrift 4025 255 (1969)). Auch diese Reduktionsverfahren sind wegen der hohen Kosten für die eingesetzten teuren Reduktionsmittel Phosphor bzw. Iod, Iodid und Iodwasserstoffsäure wirtschaftlich nachteilig. Diaryldisulfide werden weiterhin durch Reduktion von Arylsulfochloriden mit Zink erhalten (J. Chem. Soc. 123, 2384; Acta Chim. Acad. Sci. Hung. 1, 319 (1951). Hierbei fallen jedoch ökologisch sehr ungünstige Abwässer an, deren Aufbereitung hohe Kosten verursacht.

Es ist bereits bekannt (US-Patentschrift 2 571 740) daß bei der Reduktion von 3-Nitrobenzolsulfochlorid mit Iodwasserstoffsäure zu 3,3'-Dinitrodiphenyl-disulfid der Bedarf an Iodwasserstoffsäure herabgesetzt werden kann, wenn die Reduktion in Wasser oder in mit Wasser mischbaren Lösungsmitteln, beispielsweise Eisessig, Dioxan oder Methylethylketon in Gegenwart von Alkalisulfiten oder Schwefeldioxid durchgeführt wird. Bei diesem Verfahren müssen, soweit in Wasser als Lösungsmittel gearbeitet wird, immer noch pro mol 3-Nitrobenzolsulfochlorid 0,2 bis 0,5 mol HI eingesetzt werden. Im Falle des Einsatzes organischer, mit Wasser mischbarer Lösungsmittel kann die Iodwasserstoffmenge reduziert werden, jedoch ergeben sich hierbei nach der Isolierung des 3,3'-Dinitrodiphenyl-disulfids ökologisch ungünstige, organische Lösungsmittel enthaltende Abwässer.

Es ist auch bekannt (US-Patentschrift 2 986 581), Arylsulfochloride mit phosphoriger Säure und Kaliumiodid zu den entsprechenden Diaryl-disulfiden zu reduzieren, wobei als Lösungsmittel Essigsäure oder Wasser/Toluol-Gemische verwendet werden. Auch nach diesem Verfahren kann lediglich 3,3'-Dinitrodiphenyl-disulfid mit guten Ausbeuten hergestellt werden, während bei der Reduktion von Benzolsulfochlorid, p-Toluolsulfochlorid bzw. p-Chlorbenzolsulfochlorid schlechte Ausbeuten an den entsprechenden Diaryldisulfiden, verbunden mit ökologisch ungünstigen Abwässern, erhalten werden.

Überraschenderweise wurde nun gefunden, daß man aromatische Disulfide, heteroaromatische Disulfide und Farbstoffdisulfide, die nicht durch Nitrogruppen substituiert sind, in hohen Reinheiten, hohen Ausbeuten und bei geringem Verbrauch an teuren Reduktionsmitteln durch Reduktion der entsprechenden Sulfochloride mit Iodwasserstoffsäure oder mit Iodwasserstoffsäure liefernden Substanzen in Gegenwart von Reduktionsmitteln, die Iod in Iodwasserstoff überführen, herstellen kann, wenn die Reaktion in einem aus Wasser und einer organischen Phase bestehenden 2-Phasensystem in Gegenwart eines Phasentransferkatalysators durchgeführt wird.

Die als Ausgangsprodukte benötigten aromatischen Sulfochloride, heteroaromatischen Sulfochloride oder Farbstoff-Sulfochloride enthalten im Molekül eine Sulfochloridgruppe oder mehrere, z. B. bis zu vier, Sulfochloridgruppen.

Geeignete aromatische Sulfochloride leiten sich z. B. ab von Benzol, Naphthalin, Inden, Diphenyl, Acenaphthen, Fluoren, Phenanthren, Anthracen, Benzanthracen, Fluoranthren, Naphthacen, Pyren, Chrysen. Geeignete heteroaromatische Sulfochloride leiten sich z. B. ab von Heteroaromaten mit 5- oder 6-gliedrigen aromatischen Ringen mit 1, 2 oder 3 gleichen oder verschiedenen Heteroatomen, wie Sauerstoff, Schwefel und Stickstoff, wobei die heterocyclischen Ringe auch mit einem oder mehreren Benzolkernen kondensiert sein können. Geeignete Heterocyclen sind z. B. Furan, Pyrrol, Thiophen, Thiazol, Oxazol, Isoxazol, Pyrazol, Imidazol, Pyridin, Benzofuran, Dibenzofuran, Indol, Indazol, Benzimidazol, Cumaron, Carbazol, Chinolin, Pyrimidin, Phthalazin, Chinoxalin, Acridin, Phenanthridin, Phenazin.

Die aromatischen und heteroaromatischen Sulfochloride können einen oder mehrere beliebige gleiche oder verschiedene Substituenten mit Ausnahme von Nitrogruppen tragen, wie z. B. Alkyl, beispielsweise mit 1 bis 10 C-Atomen, insbesondere mit 1 bis 8 C-Atomen, Alkoxy, beispielsweise mit 1 bis 10 C-Atomen insbesondere mit 1 bis 6 C-Atomen, Hydroxy, Cyan, Halogen, insbesondere Chlor, Fluor, Brom, Carboxy, Alkoxycarbonyl, insbesondere mit 2 bis 6 C-Atomen Phenyl, Alkylcarbonylamino, insbesondere mit 2 bis 6 C-Atomen Alkylthio insbesondere mit 1 bis 5 C-Atomen, Trifluormethyl.

Für das erfindungsgemäße Verfahren sind als Ausgangsprodukte beispielsweise folgende aromatische Sulfochloride geeignet: Benzol-sulfochlorid, 2-, 3- und 4-Toluol-sulfochlorid, 2-, 3- und 4-Chlorbenzol-sulfochlorid,

2-, 3- und 4-Brombenzol-sulfochlorid, 4-Fluorbenzol-sulfochlorid, 2-, 3- und 4-Methoxybenzol-sulfochlorid, 2-, 3- und 4-Ethoxybenzol-sulfochlorid, 4-Butoxybenzol-sulfochlorid, 3-Carboxybenzol-sulfochlorid, 4-Carboxybenzol-sulfochlorid, 3-Carboxy-4-chlorbenzol-sulfochlorid, 4-Carboxy-2-chlorbenzol-sulfochlorid, 2-Methoxycarbonylbenzol-sulfochlorid, 4-Methoxycarbonylbenzol-sulfochlorid, 2,4- 2,5- und 2,6-Dichlorbenzol-sulfochlorid, 2,4,6-Trichlor-sulfochlorid, 2,4-Dichlor-5-methylbenzol-sulfochlorid, 4-Chlor-2,5-dimethylbenzol-sulfochlorid, 2-Cyan-5-ethoxybenzol-sulfochlorid, 2-Cyan-3-methyl-5-chlorbenzol-sulfochlorid, 4-Acetylaminobenzol-sulfochlorid, 3-Hydroxybenzol-sulfochlorid, 3-Chlor-6-ethylthiobenzol-sulfochlorid, Diphenyl-4-sulfochlorid, 3-Carboxy-4-hydroxybenzol-sulfochlorid, 2,3,4,5-Tetrachlorbenzol-sulfochlorid, 3-Trifluormethylbenzol-sulfochlorid, 4-Octylbenzol-sulfochlorid, 2-Acetylamino-4-methoxybenzol-sulfochlorid, 4-Methylthiobenzol-sulfochlorid, Naphthalin-1-sulfochlorid, Naphthalin-2-sulfochlorid, 8-Chlornaphthalin-1-sulfochlorid, 2-Methoxynaphthalin-1-sulfochlorid, 6-Hydroxynaphthalin-2-sulfochlorid, Pyren-1-sulfochlorid, Anthrachinon-1-sulfochlorid, 1-Amino-4-brom-anthrachinon-2-sulfochlorid.

Ferner sind als Ausgangsprodukte beispielsweise folgende heteroaromatische Sulfochloride geeignet: Thiophen-2-sulfochlorid, Thiophen-3-sulfochlorid, Carbazol-3-sulfochlorid, N-Ethylcarbazol-3-sulfochlorid, Pyridin-3-sulfochlorid, Chinolin-8-sulfochlorid, 8-Hydroxychinolin-5-sulfochlorid, 5,7-Dichlorchinolin-8-sulfochlorid, Dibenzofuran-2-sulfochlorid5-Phenyl-1,3,4-triazol-2-sulfochlorid.

Die als Ausgangsprodukte eingesetzten aromatischen und heteroaromatischen Sulfochloride besitzen vorzugsweise eine Sulfochloridgruppe im Molekül ($RSO_2Cl$ ). Sie werden nach dem erfindungsgemäßen Verfahren in die entsprechenden Disulfide (R-S-S-R) überführt. Aromatische und heteroaromatische Disulfochloride ($ClO_2SRSO_2Cl$), beispielsweise Benzol-1,3-disulfochlorid, Naphthalin-1,5-disulfochlorid, 2,5-Dichlorbenzol-1,3-disulfochlorid, Diphenyl-4,4'-disulfochlorid, Diphenylsulfid-4,4'-disulfochlorid, Diphenylsulfon-3,3'-disulfochlorid, 4,4'-Dichlordiphenylsulfon-3,3'-disulfochlorid werden nach dem erfindungsgemäßen Verfahren in die entsprechenden polymeren Disulfide überführt.

Als Ausgangsprodukte geeignete Farbstoff-sulfochloride sind beispielsweise Monosulfochloride, insbesondere aber Di-, Tri- und Tetrasulfochloride des Phthalocyanins, Kupferphthalocyanins, Nickelphthalocyanins, Kobaltphthalocyanins wie z. B. Kupferphthalocyanin-disulfochlorid, Kupferphthalocyanin-trisulfochlorid, Kupferphthalocyanin-tetrasulfochlorid, Nickelphthalocyanin-tetrasulfochlorid, Phthalocyanin-tetrasulfochlorid, Kobaltphthalocyanintrisulfochlorid, sowie sulfochloridgruppenhaltige Dioxazine, wie z. B. Tetra-sulfochlorid von Cl Pigment Violet 23, Di-, Tri- bzw. Tetra-sulfochlorid von 6,13-Dichlortriphenodioxazin; 3-Chlorsulfonylphenylazo-1-(2-chloro-4-chlorsulfonyl-6-methylphenyl)-3-methylpyrazolon(5).

Die für das erfindungsgemäße Verfahren als Ausgangsprodukte benötigten aromatischen, heteroaromatischen oder Farbstoff-Sulfochloride sind bekannte Verbindungen bzw. können nach literaturbekannten Verfahren hergestellt werden, beispielsweise a) durch direkte Einführung der Sulfochlorid-Gruppe in Aromaten, Heterocyclen oder in wasserunlösliche Farbstoffe bzw. Pigmente (vgl. beispielsweise Ullmanns Enzyklopädie der technischen Chemie, Band 8, 4. Auflage, Seite 417 (1974); Houben-Weyl, Methoden der Organischen Chemie, Band IX, 4. Auflage, Seite 572 (1955); K. Venkataraman, The Chemistry of Synthetic Dyes, Vol. VII, Seiten 12 bis 15 (1974) und die jeweils dort zitierte Literatur); b) durch Reaktion von Aryl- bzw. Hetarylsulfonsäuren oder deren Natriumsalzen mit beispielsweise Phosphorpentachlorid oder Thionylchlorid (vgl. z. B. Houben-Weyl, Methoden der Organischen Chemie, 4. Auflage, Band IX, Seite 563 (1955); Helv. Chim. Acta 42, 1654 (1959); c) aus aromatischen Aminen über die entsprechenden Diazoniumsalze (vgl. beispielsweise H. Meerwein et al, Chem. Ber. 90, 841 (1957)).

Das erfindungsgemäße Verfahren wird in einem 2-Phasensystem durchgeführt, das aus einer organischen Phase und einer wäßrigen Phase besteht. Die organische Phase wird dabei aus einem organischen Lösungsmittel gebildet, das in Wasser nicht oder nur wenig löslich ist und das nach Beendigung des Verfahrens wieder leicht regeneriert werden kann, z. B. durch azeotrope Destillation oder durch Wasserdampfdestillation. Als geeignete organische Lösungemittel kommen beispielsweise in Betracht: aliphatische Kohlenwasserstoffe, wie z. B. Hexan, Cyclohexan, Methylcyclohexan; aromatische Kohlenwasserstoffe, wie z. B. Benzol, Toluol, o-, m-, p-Xylol; teil- und vollhalogenierte Kohlenwasserstoffe, wie z. B. Methylenchlorid, Chloroform, Tetrachlormethan, 1,1,2-Trichlorethan, Trichlorethylen, Ethylenchlorid, 1-Chloroctan, Chlorbenzol, o-Dichlorbenzol, cis-1,2-Dichlorethylen, 1,1,1,2-Tetrachlorethan, Tetrachlorethylen, 1,1,2,2,-Tetrachlorethan, 1,1,2-Trichlortrifluorethan; Ketone, wie z. B. Methylethylketon, Methyl-n-propylketon, Diethylketon, 2-Hexanon, 3-Hexanon, Di-n-propylketon, Benzophenon; Nitrile, wie z. B. Acetonitril, 1-Cyanooctan, Benzonitril; Ether, wie z. B. Diethylether, Ethyl-propylether, Di-n-butylether; Ester, wie z. B. Ethylacetat, Butylacetat; Nitroverbindungen, wie z. B. Nitromethan, Nitrobenzol.

Die organische Phase kann auch aus einem Gemisch verschiedener organischer Lösungsmittel bestehen. Falls das einzusetzende Sulfochlorid einen niedrigen Schmelzpunkt aufweist und bei der benötigten Reaktionstemperatur in flüssiger Form vorliegt, kann auch das Sulfochlorid als organische Phase dienen.

Geeignete Phasentransferkatalysatoren sind beispielsweise beschrieben in Charles M. Starks and Charles Liotta: Phase Transfer Catalysis, Academic Press, New York, San Francisco, London, (1978), 57 bis 88; F. Vögtle (Editor), Host Guest Complex Chemistry I, Springer Verlag Berlin, Heidelberg, New York, (1981), 3 bis 16, erschienen in der Reihe "Topics in Current Chemistry" als Band 98; E.V. Dehmlov in Angewandte Chemie, Internationale Edition, Band 13, Nr. 3, (1974), 170; Jozef Dockx in Synthesis (1973), 441 bis 456; Herbert Lehmkuhl, Farroch Rabet, Klaus Hauschild in Synthesis, (1977), 184 bis 186; Fernando Montanari , Dario Landini, Franco Rolla: Phase Transfer Catalyzed Reactions in Topics in Current Chemistry (1982), Vol. 101, 163 bis 172.

Im Rahmen der vorliegenden Erfindung sind als Phasentransferkatalysatoren besonders geeignet Oniumsalze, z. B. solche der Formeln

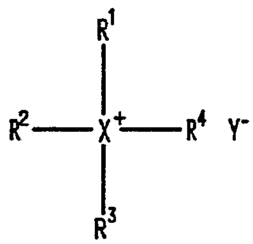

(Ia)

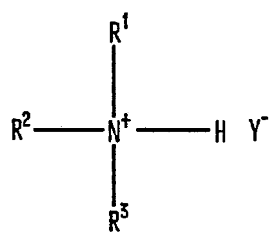

(Ib)

worin $R^1$, $R^2$, $R^3$, $R^4$ gleiche oder verschiedene Alkylreste mit 1 bis 25 C-Atomen, Hydroxyalkylreste mit 2 bis 25 C-Atomen, Arylreste, insbesondere Phenyl, Aralkylreste, insbesondere Benzyl, Cycloalkenylreste, insbesondere Cyclopentenyl und Cyclohexenyl und $X^+ = N^+$, $P^+$, $As^+$ oder $S^+$ und $Y^- =$ ein Anion, insbesondere $Cl^-$, $Br^-$, $I^-$, $NO_3^-$ $HSO_4^-$, 1/2 $SO_4^{=}$, 1/3 $PO_4^{\equiv}$, $CN^-$ , Tosylat, Benzoat, Picrat, p-Nitrobenzoat, Perchlorat, β-Naphthalin-sulfonat, Acetat bedeuten. In den Formeln Ia und Ib können auch zwei der Reste $R^1$, $R^2$, $R^3$ oder $R^4$ zusammen mit dem $X^+$ oder $N^+$, an das sie gebunden sind, ein Ringsystem mit 5 bis 7 C-Atomen bilden. Von den Verbindungen der Formel Ia sind Ammoniumsalze bevorzugt. Die Verbindungen der Formel Ib können auch in Form der tertiären Amine eingesetzt werden. Die Oniumsalze Ib bilden sich dann unter den Reaktionsbedingungen. Auch Iodoniumsalze, die sinngemäß den Oniumverbindungen der Formel Ia oder Ib entsprechen, sind geeignet.

Auch Di- und Poly-oniumsalze sind geeignet, das sind solche Verbindungen, in denen die Oniumgruppierung, z. B. $R^1$, $R^2$, $R^3$, $R^4$ , $X^+$ , mehrere Male wiederholt ist. Dabei sind die $X^+$-Atome durch Aralkylgruppen oder lange Alkylreste mit mehr als 10 C-Atomen miteinander verknüpft.

Die Oniumsalze enthalten vorzugsweise mehr als 10 C-Atome, vorzugsweise 16 bis 40 C-Atome, in der Regel aber weniger als 70 C-Atome, pro positiv geladenem Oniumatom.

Ferner sind als Phasentransferkatalysatoren geeignet: cyclische Oligomere des Ethylenoxids oder makrocyclische Polyether, insbesondere solche der Formel II

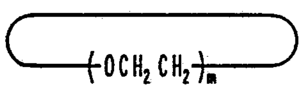

(II)

wobei m eine ganze Zahl von 4 bis 12, insbesondere 4 bis 10, bedeutet, das sind sogenannte Kronenether, an die auch ein oder mehrere Benzol- und/oder Cyclohexankerne ankondensiert sein können (C.J.Pederson, J.A.C.S. 89, (1967) 7017); ferner Coronandsulfide (Thiakronenether), das sind Kronenether der vorgenannten Formel II, bei denen ein Sauerstoffatom, mehrere oder alle Sauerstoffatome durch -S- ersetzt sind; Coronandamine (Azakronenether), das sind Kronenether der vorgenannten Formel II, bei denen ein Sauerstoffatom, mehrere oder alle Sauerstoffatome durch -NH-Gruppen ersetzt sind. Entsprechend sind auch gemischte O, N, S, P-Coronanden, das sind Kronenether der Formel II, bei denen ein Sauerstoffatom, mehrere oder alle Sauerstoffatome durch -NH-, -S- und/oder phosphorhaltige Gruppen, wie z. B. $-P(O)CH_3-$ ersetzt sind, sowie Coronanden mit heteroaromatischen Ringen geeignet, das sind Kronenether der Formel 11 bzw. gemischte O, N, S-Coronanden, bei denen eine, mehrere oder alle Ethanopositionen ($-CH_2OCH_2-$) durch in den Kronenetherring inkorporierte gleiche oder verschiedene heteroaromatische Kerne, wie Furan, Pyridin und/oder Thiophen, d.h. durch Furan-2,5-diyl, Pyridin-2,6-diyl und/oder Thien-2,5-diyl, ersetzt sind. Ferner sind z. B. geeignet Cryptanden, das sind Azakronenether oder Coronandamine mit mindestens zwei Stickstoffatomen im Kronenetherring, die durch zusätzliche Oligoetherketten, in denen ein, mehrere oder alle Sauerstoffatome auch durch -NH- und/oder -S-Gruppen ersetzt sein können, überbrückt sind, wodurch bi-, tri- und polycyclische Systeme entstehen, sowie mehrseitige Kronenverbindungen. An alle vorgenannten Verbindungen können auch eine oder mehrere Benzol- oder Cyclohexankerne ankondensiert sein. Gut geeignet sind auch offenkettige Kronenverbindungen sowie offenkettige Cryptanden, die man beide auch als sogenannte Podanden bezeichnet. Geeignete offenkettige Kronenverbindungen sind insbesondere acyclische Oligomere des Ethylenoxids, vor allem solche der Formeln

$$R^5-(OCH_2CH_2)_p-O-R^6 \quad (III)$$

$$R^5-O-(CH_2CH_2O)_p-(CH_2)_q-CH_2O-R^6 \quad (IV)$$

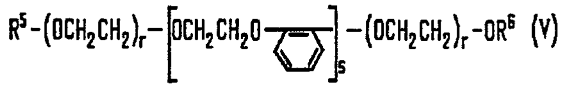 (V)

worin $R^5$ $R^6$ verschieden oder vorzugsweise gleich sind und Alkylreste mit 1 bis 15 C-Atomen, insbesondere Methyl; Chinolyl, insbesondere Chinolyl-8; Alkylcarbonyl mit 1 bis 15 C-Atomen im Alkylrest, insbesondere Methylcarbonyl; Phenyl; Phenyl substituiert, insbesondere in 2-Stellung, durch Alkoxy mit 1 bis 4 C-Atomen, insbesondere $OCH_3$, Nitro, COOH, Alkoxy-carbonyl mit 1 bis 4 C-Atomen im Alkoxyrest, insbesondere $COOC_2H_5$, Alkylaminocarbonyl mit 1 bis 4 C-Atomen im Alkylrest, Alkylcarbonylamino mit 1 bis 4 C-Atomen im Alkylrest und p eine ganze Zahl von 3 bis 50, insbesondere eine ganze Zahl von 4 bis 25, q eine ganze Zahl von 1 bis 10, insbesondere 3, r eine ganze Zahl von 2 bis 12 und s eine ganze Zahl von 1 bis 6, insbesondere eine ganze Zahl von 1 bis 4, bedeuten. Ferner sind als Phasentransferkatalysatoren geeignet sogenannte Podandocoronanden, Oktopusmoleküle und makrocyclische Oligoketone, Oligothioketone und Spheranden. Geeignete makrocyclische Oligoketone oder Oligothioketone leiten sich von der oben angegebenen Kronenetherformel durch Ersatz eines Sauerstoffatoms, mehrerer oder aller Sauerstoffatome durch Ketogruppen (-CO-) und/oder Thioketogruppen (-CS-) ab. Die vorstehend aufgeführten Phasentransferkatalysatoren sind insbesondere z. B. beschrieben in F. Vögtle, Host Guest Complex Chemistry I, loc.cit., und in der dort zitierten Literatur.

Im Rahmen des erfindungsgemäßen Verfahren sind als Phasentransferkatalysatoren ferner geeignet tertiäre Amine der Formel VI

$$N-\left[CHR^7-CHR^8-O-(CHR^9-CHR^{10}-O)_t-R^{11}\right]_3 \quad (VI)$$

worin t eine ganze Zahl von 0 bis 10 ($0 \leq n \leq 10$) ist, $R^7$, $R^8$, $R^9$ und $R^{10}$ gleich oder verschieden sind und ein Wasserstoffatom oder einen Alkyl- oder Cycloalkylrest mit 1 bis 12 C-Atomen, einen Phenylrest oder einen $C_vH_{2v+1}-C_6H_4-$ oder $-C_vH_{2v}-C_6H_5$ Rest bedeuten, in dem v eine ganze Zahl von 1 bis 12 bedeutet. Die Herstellung der Verbindungen der Formel VI ist in den europäischen Patentschriften 21 868 und 21 927 beschrieben.

Beispiele für geeignete Oniumsalze der Formel Ia und Ib sind: Tributylamin, Tetrabutylammonium-chlorid, -bromid, -iodid, -acetat, -sulfat; Benzyl-tri-butylammonium-chlorid, -bromid, -iodid; Trialkyl-methylammonium-chlorid, -bromid, -iodid, vorzugsweise mit 7 bis 12 C-Atomen in den Alkylresten, wie z. B. Trioctyl-methylammonium-chlorid; Dialkyl-dimethylammonium-chlorid, -bromid bzw. -iodid, vorzugsweise mit 8 bis 20 C-Atomen in den Alkylresten, wie z. B. Dihexadecyl-dimethylammonium-bromid, Dilauryl-dimethylammonium-chlorid, Dieicosyl-dimethylammonium-chlorid; Alkyl-trimethylammonium-chlorid, -bromid bzw. -iodid, vorzugsweise mit 10 bis 25 C-Atomen im Alkylrest, wie z. B. Hexadecyl-trimethylammonium-bromid, Octyl-tributylammoniumbromid; Alkyl-dimethylbenzylammonium-chlorid, -bromid bzw. -iodid, vorzugsweise mit 10 bis 20 C-Atomen im Alkylrest; Hexadecylpyridinium-bromid; Tetradodecylammonium-bromid; Tetraheptylammonium-bromid; Tetrahexylammonium-chlorid, -bromid bzw.-iodid; Tributylheptylammonium-bromid; N-Benzyl-triethylammonium-chlorid; Ethyl-tri-octylphosphonium-bromid; Tetrabutyl-phosphonium-bromid, Hexadecyl-tripropylphosphonium-bromid,Triphenyl-ethylphosphonium-bromid;3-Benzyl-5(2-hydroxyethyl)-4-methyl-thiazolium-chlorid; Trialkylammonium-chlorid, -bromid bzw. -iodid; insbesondere mit 7 bis 12 C-Atomen in den Alkylresten; Dialkyl-methylammonium-chlorid, -bromid, -iodid, insbesondore mit 8 bis 20 C-Atomen in den Alkylresten; Alkyl-dimethylammonium-chlorid, -bromid bzw. -iodid, insbesondere mit 10 bis 25 C-Atomen im Alkylrest. Auch Gemische verschiedener Oniumsalze können verwendet werden.

Beispiele für weitere geeignete Phasentransferkatalysatoren sind: 12-Krone-4, 15-Krone-5, 18-Krone-6, 21-Krone-7, 24-Krone-8, Benzo-15-krone-5, Dibenzo-18-krone-6, Dicyclohexano-18-krone-6, Dibenzo-24-krone-8, Dicyclohexano-24-krone-8, Dibenzo-30-krone-10, [1,1]Diazakrone, [2,1]Diazakrone, [2,2]Diazakrone,[3,2]Diazakrone, [3,3]-Diazakrone, 5,6,14,15-Dibenzo-8,12-diaza-1,4-dioxacyclopentadeca-5,14-dien, 4,7,13,16,21,24-Hexaoxa-1,10-diazabicyclo[8,8,8]-hexacosan (=[2,2,2]Cryptand), [1,1,1,]Cryptand, [2,1,1]Cryptand, [2,2,1]Cryptand, [3,3,3]Cryptand, {3}<N[ONO]$_2$[O]N<[O]$^{7,19}$-Cryptand-10>, 1,16-Dimethyl($O_6$-podand-6) (=Pentaglyme), Tris(3-oxa-butyl)amin, Tris(3,6,9-trioxa-decyl)amin, Tris(3,6-dioxa-heptyl)amin, Tris(3,6-dioxa-octyl)amin, Tris(3,6,9-trioxa-undecyl)amin, Tris(3,6-dioxa-nonyl)amin, Tris-(3,6,9-trioxa-dodecyl)amin, Tris(3,6,9-trioxa-tridecyl)amin, Tris(3,6,9,12,15,18-hexaoxa-nonadecyl)amin, Tris(3,6-dioxa-4-methyl-heptyl)amin, Hepta-ethylenglykol-dimethylether,Docoso-ethylenglykol-dimethylether,Pentatetraconta-ethylenglykol-dimethylether, Octa-ethylenglykol-methyl-dodecyl-ether, Octa-ethylenglykol-diphenyl-ether, Octa-ethylenglykol-methylether-acetat (=$H_3CO-(CH_2CH_2O)_8-COCH_3$). Auch Gemische sowie auch technische Gemische verschiedener Phasentransferkatalysatoren können verwendet werden.

Als eigentliches Reduktionsmittel wird Iodwasserstoffsäure eingesetzt. Anstelle von Iodwasserstoffsäure kann auch eine Iodwasserstoffsäure liefernde Substanz, wie z. B. ein Iodid, insbesondere ein Alkaliiodid, wie Natrium- oder Kaliumiodid, Phosphortriiodid oder elementares Iod verwendet werden. Zur Reduktion einer Sulfochloridgruppe in einem mol Ausgangsverbindung werden 0,001 bis 0,5 mol, vorzugsweise 0,005 bis 0,1 mol Iodwasserstoffsäure oder Iodwasserstoffsäure liefernde Substanz verwendet. Ferner wird noch ein weiteres Reduktionsmittel zugesetzt, welches das gegebenenfalls eingesetzte Iod und das bei der Reduktion frei werdende Iod unter den Reaktionsbedingungen in Iodwasserstoffsäure überführt. Reduktionsmittel mit einem derartigen Redoxpotential sind z. B. Schwefeldioxid, Schwefeldioxid liefernde Substanzen, wie z. B. Hydrogensulfite, Sulfite oder Pyrosulfite; ferner sind z. B. geeignet: phosphorige Säure, Phosphite, hypophosphorige Säure, Hypophosphite, Hydrazin, Hydrazinsalze, wie Hydrazin-chlorid, Hydrazinsulfat oder Dihydrazin-sulfat. Als Hydrogensulfite, Sulfite, Phosphite, Hypophosphite, Pyrosulfite werden vor allem die entsprechenden Alkalisalze, insbesondere die entsprechenden Natrium- oder Kaliumsalze, eingesetzt. Es können auch Gemische verschiedener derartiger Reduktionsmittel zur Anwendung kommen, sofern keine Nebenreaktionen zwischen ihnen eintreten.

Bei den oben genannten Mengen an Iodwasserstoffsäure oder einer Iodwasserstoffsäure liefernden Substanz werden 1 bis 2, vorzugsweise 1,25 bis 1,75, Reduktionsäquivalente des Reduktionsmittels benötigt, das Iod in Iodwasserstoffsäure überführt. Das sind bei Schwefeldioxid, Hydrogensulfit, Sulfit, phosphorige Säure und Phosphit 2 bis 4 mol, vorzugsweise 2,5 bis 3,5 mol; bei hypophosphoriger Säure, Hypophosphit, Pyrosulfit, Hydrazin, Hydrazinchlorid, Hydrazin-sulfat sind das 1 bis 2 mol, vorzugsweise 1,25 bis 1,75 mol, und bei Dihydrazin-sulfat sind das 0,5 bis 1 mol, vorzugsweise 0,625 bis 0,875 mol.

Pro mol Ausgangsverbindung werden normalerweise 0,0001 bis 0,05 mol, vorzugsweise 0,001 bis 0,02 mol des Phasentransferkatalysators eingesetzt.

Das erfindungsgemäße Verfahren wird bei Temperaturen von 20 bis 200°C, vorzugsweise 40 bis 150°C, unter Rühren durchgeführt. Bei Reaktionstemperaturen, die unter Normaldruck oberhalb des Siedepunkts einer Komponente des 2-Phasensystems liegen, wird die Reaktion unter dem sich einstellenden Überdruck in einem Autoklaven ausgeführt. Auch wenn als weiteres Reduktionsmittel Schwefeldioxid oder eine Schwefeldioxid liefernde Substanz verwendet wird, ist die Durchführung der Reaktion in einem Autoklaven zweckmäßig. Sie ist in dem genannten Temperaturbereich normalerweise nach Reaktionszeiten von 15 Minuten bis 6 Stunden beendet. Zu Beginn der Reaktion sollte die wäßrige Phase des 2-Phasensystems einen pH-Wert kleiner als 4 besitzen. Normalerweise stellt sich ein derartiger pH-Wert in der wäßrigen Phase ohne besonderes Zutun ein. Nur für den Fall, daß für die Reduktion des Iods zu Iodwasserstoffsäure ein stark alkalisches Reduktionsmittel, wie Hydrazin, verwendet wird, ist eine entsprechende gesonderte Einstellung des pH-Werts, z. B. mit einer Mineralsäure, wie HCl, $H_2SO_4$ etc., erforderlich. Nach Beendigung der Umsetzung wird das gegebenenfalls eingesetzte organische Lösungsmittel durch azeotrope oder Wasserdampf-Destillation entfernt. Danach können in den meisten Fällen die Endprodukte, das heißt z. B. die Diaryl- bzw. Dihetaryldisulfide oder die disulfidgruppenhaltigen Farbstoffe durch einfache Filtration aus dem wäßrigen Reaktionsmedium isoliert werden. Aufgrund des hohen Reinheitsgrads der zumeist in hohen bis praktisch quantitativen Ausbeuten anfallenden Produkte ist eine weitere Reinigungsoperation normalerweise nicht erforderlich.

Um die Endprodukte in einer besserfiltrierbaren Form zu erhalten, wird dem Reaktionsansatz nach beendeter Reduktion zweckmäßigerweise ein Tensid zugesetzt. Geeignete Tenside sind z. B. Aniontenside, wie z. B. Schwefelsäureester, wie sulfatierte Öle und Fettsäuren, sulfatierte Ester und Amide, Alkylsulfate, sulfatierte Ether, ferner Alkylsulfonate, wie Sulfobernsteinsäureester, Alkylnaphthalinsulfonate; Kationtenside, wie primäre, sekundäre und tertiäre Aminsalze, quartäre Ammonium-, Phosphonium- oder Sulfoniumverbindungen, Amphotenside, wie Betaine, Sulfobetaine und Sulfatbetaine; nichtionogene Tenside, wie z. B. Fettsäureester von Alkoholen, Ethylenglykol, Polyethylenglykolen, Propylenglykolen, Glycerin, Polyglycerin, Sorbit, Pentaerythrit, Saccharose; Fettamine, Fettamide, Polyamine, ferner Polyglykolether von Alkoholen, Fettsäuren, Fettsäureestern, wie Glyceriden oder Sorbitestern, Fettaminen, Polypropylenglykolen, Alkylphenolen; ferner Polypropylenglykolether von Alkoholen, Fettsäuren, Fettaminen. Geeignete Tenside sind z. B. beschrieben in Ullmanns Enzyklopädie der techn. Chemie, 3. Auflage, Bd. 16 (1965), 724 bis 736, und J.P.Sisley, Encyclopedia of Surface-Active Agent, Chemical Publishing Comp. Inc., New York (1964). Bevorzugte Tenside sind Ethoxylierungsprodukte von Fettalkoholen, Fetten, Fettsäuren sowie Alkansulfonate.

Es können auch Gemische oder gegebenenfalls technische Gemische von Tensiden verwendet werden. Von dem Tensid werden normalerweise pro mol Ausgangsverbindung der Formel I 0,0001 bis 0,05 mol, vorzugsweise 0,001 bis 0,02 mol, eingesetzt. In vielen Fällen kann das Tensid dem Reaktionsansatz auch von Beginn an zugesetzt werden.

Die nach dem erfindungsgemäßen Verfahren hergestellten disulfidgruppenhaltigen Farbstoffe stellen wasserunlösliche Schwefelfarbstoffe dar. Diese können nach an sich bekannten Methoden (vgl. beispielsweise Ch. Heid et al, Melliand Textber. 12, 1314 (1973)) in die wasserlöslichen Leuko-Schwefelfarbstoffe überführt werden, die sich hervorragend zum Färben von Cellulosefasern eignen. Die Schwefelfarbstoffe weisen eine hohe Farbstärke und ein hohes Echtheitsniveau auf.

Das erfindungsgemäße Verfahren weist unter anderem den Vorteil auf, daß unsubstituierte und mit Ausnahme von nitrosubstituierten beliebig substituierte Aryl- und Hetarylsulfochloride bzw. sulfochloridgruppenhaltige Farbstoffe mit sehr guten Ausbeuten in die entsprechenden Disulfide überführt werden können. Die

erhaltenen Disulfide weisen einen hohen Reinheitsgrad auf. Die erfindungsgemäße Reduktionsmethode ist nicht auf die Herstellung von 3,3'-Dinitrodiphenyl-disulfid aus leicht reduzierbarem 3-Nitrophenylsulfochlorid eingeschränkt. Ausbeutemindernde Nebenreaktionen, beispielsweise beschrieben in Houben-Weyl, Methoden der Organischen Chemie, Bd. IX, Seiten 30 bis 31 (1955); J. Chem. Soc. 125, 2359 (1924); Ber. dtsch. Chem. Ges. 24, 335 (1891) treten überraschenderweise nicht auf. Die nach der Produktisolierung anfallenden Abwässer sind ökologisch günstig. Die Wirksamkeit von Phasentransferkatalysatoren in dem erfindungsgemäßen Verfahren ist besonders überraschend und war auch für den Fachmann nicht naheliegend, nachdem aus Chem. Ber. 112, 2765 (1979) bereits bekannt war, daß Iodwasserstoffsäure durch Phasentransferkatalysatoren nicht in ein organisches Medium extrahiert wird.

Die folgenden Beispiele dienen zur weiteren Erläuterung der Erfindung. Angegebene Prozente bedeuten, soweit nichts anderes angegeben ist, Gewichtsprozente.

**Beispiel 1**

Eine in bekannter Weise aus 49,8 g Phosphortrichlorid und 25,8 g Chlor in 140 ml Monochlorbenzol hergestellte Suspension von Phosphorpentachlorid wird mit 79,8 g 8-Chlornaphthalin-1-sulfonsaurem Natrium versetzt. Anschließend rührt man zur Vervollständigung der Reaktion mehrere Stunden bei 60 bis 80°C nach und kühlt dann auf 20°C. Darauf wird die Lösung von 8-Chlornaphthalin-1-sulfochlorid unter Außenkühlung mit 300 ml Wasser versetzt. Es werden 1,9 ml Iodwasserstoffsäure 57 %ig und 2,7 g einer 50 %igen Chlorbenzollösung eines handelsüblichen Tricaprylylmethylammonium-chlorid-phasentransferkatalysators (Caprylyl ist dabei eine Mischung von $C_8H_{17}$ bis $C_{10}H_{21}$ Alkylgruppen) zugefügt. Man heizt das Gemisch unter Rühren auf 40°C und gibt im Verlauf von 45 Minuten eine Lösung von 68,4 g Natriumpyrosulfit in 140 ml Wasser zu. Nach einer Reaktionszeit von 2 Stunden bei 70°C ist die Reduktion beendet. Man entfernt Chlorbenzol aus dem Reaktionsgemisch durch Wasserdampfdestillation, kühlt die wäßrige Suspension auf ca. 40°C, filtriert und wäscht das Produkt mit Wasser säurefrei.

Nach Trocknung bei 80 bis 90°C erhält man 58,7 g (99 % der Theorie) Bis-(8-chlornaphthyl-1)-disulfid mit einem Reinheitsgrad von 97 %; Fp. 173 bis 175°C.

**Vergleichsbeispiel 1**

Die Reduktion wird nach den Angaben des Beispiels 1, jedoch ohne Zusatz des Phasentransferkatalysators, durchgeführt. Chromatographisch ist nach einer Reaktionszeit von 2 h bei 70°C kein Bis-(8-chlornaphthyl-1)-disulfid nachzuweisen. Die Chlorbenzolphase enthält noch 10,4 g 8-Chlornaphthalin-1-sulfochlorid.

**Beispiel 2**

Die Herstellung einer Lösung von 8-Chlornaphthalin-1-sulfochlorid in Chlorbenzol, ausgehend von 79,4 g 8-chlornaphthalin-1-sulfonsaurem Natrium, erfolgt nach den Angaben des Beispiels 1. Anschließend setzt man 1,9 g Iod und 2,7 g des in Beispiel 1 benutzten Phasentransferkatalysators zu. Die Mischung wird in einen 1,3 l Tantal-Autoklaven überführt und bei 20°C mit einer Lösung von 78,4 g Natriumpyrosulfit in 150 ml Wasser versetzt. Anschließend heizt man auf 100°C und rührt 5 Stunden bei 100°C bis zur Druckkonstanz von 2 bar nach. Die Produktisolierung erfolgt nach den Angaben des Beispiels 1.

Ausbeute: 58,7 g (97 % der Theorie) Bis-(8-chlornaphthyl-1)-disulfid; Reinheitsgrad: 96 %; Fp. 172 bis 174°C.

**Beispiele 3 bis 14**

Die Herstellung einer Lösung von 8-Chlornaphthalin-1-sulfochlorid aus 79,4 g (0,3 mol) 8-chlornaphthalin-1-sulfonsaurem Natrium und anschließende Wasserzugabe erfolgt nach den Angaben des Beispiels 1.

Nach Zugabe der in der nachfolgenden Tabelle I aufgeführten Zusätze werden in einem Tantal-Autoklaven 49 ml Schwefeldioxid aufgedrückt. Die Reaktionszeit beträgt 5 Stunden bei 100°C. Die Produktisolierung erfolgt nach den Angaben des Beispiels 1.

**TABELLE I**

| Beispiel Nr. | Zugesetzte Menge an Phasentransferkatalysator | HI oder Iodid | Bis-(8-chlornaphtyl-1)-disulfid Ausbeute in % d.Th. | Schmelzpunkt in °C |
|---|---|---|---|---|
| 3 | 2,7 g A 50 % in Chlorbenzol | 1,4 g NaI | 96 | 173—174 |
| 4 | 2,7 g A | 2 g KI | 97 | 172—175 |
| 5 | 1,5 g A | 0,8 g HI 57 % ig | 92 | 170—173 |
| 6 | 1,6 g B | 3,2 g HI 57 % ig | 97 | 174—176 |
| 7 | 2 g C 40 % ig Wasser | 3g HI 57 % ig | 95 | 173—195 |
| 8 | 2,3 g D 50 % ig in Wasser | 3g HI 57 % ig | 99 | 173—194 |
| 9 | 1,5 g Ethyltrioctylphosphoniumbromid | 3g HI 57 % ig | 93 | 172—175 |
| 10 | 4 g Tetrabutylammoniumiodid | — | 91 | 172—174 |
| 11 | 1,2 g Trioctylammoniumchlorid | 3 g HI 57 % ig | 90 | 173—175 |
| 12 | 1,5 g E | 3,3g HI 57 % ig | 89 | 170—172 |
| 13 | 2,0 g F | 3,3g HI 57 % ig | 91 | 170—171 |
| 14 | 2,0 g G | 3,3g HI 57 % ig | 94 | 172—173 |

In der vorstehenden Tabelle bedeuten:

A: Tricaprylylmethylammoniumchlorid (Caprylyl ist dabei eine Mischung von $C_8H_{17}$ bis $C_{10}H_{21}$-Alkylgruppen)

B: Diheptadecyl-dimethylammoniumchlorid (Heptadecyl ist dabei eine Mischung von $C_{16}H_{33}$ bis $C_{18}H_{37}$-Alkylgruppen)

C: Tridecyl-trimethyl-ammoniumchlorid (Tridecyl ist dabei eine Mischung von $C_{12}H_{25}$ bis $C_{14}H_{29}$-Alkylgruppen)

D: Tridecyl-benzyl-dimethyl-ammoniumchlorid (Tridecylbenzyl ist dabei eine Mischung von $C_{12}H_{25}$ bis $C_{14}H_{29}$-Alkylgruppen)

E: 18-Krone-6

F: 2,5,8,11,14,19-Hexaoxa-eicosan

G: Tris(3,6,9-trioxa-decyl)amin

Wie das Beispiel 10 zeigt, ist ein gesonderter Zusatz von Iodwasserstoffsäure, Iodid oder Iod nicht erforderlich, wenn der Phasentransferkatalysator ein Iodid ist.

**Beispiel 15**

77,9 g (0,3 mol) 2,4-Dichlor-5-methylbenzol-sulfochlorid, hergestellt durch Sulfochlorierung von 2,4-Dichlortoluol mit Chlorsulfonsäure und Fällung auf Eis, werden in Form des feuchten, 62,9 %igen Preßkuchens in eine Mischung von 100 ml Wasser und 100 ml Chlorbenzol eingetragen. Man setzt 2,7 g des in Beispiel 1 verwendeten Phasentransferkatalysators (50 %ig in Chlorbenzol) und 1,9 ml Iodwasserstoffsäure zu und drückt in einem 500 ml Tantal-Autoklaven 50 ml Schwefeldioxid auf. Anschließend heizt man unter Rühren auf 95 bis 100°C und rührt 5 Stunden bis zur Druckkonstanz (2 bar) nach. Darauf wird gekühlt, entspannt und das Chlorbenzol aus der Reaktionsmischung durch azeotrope Destillation entfernt. Das Produkt wird bei 20°C abfiltriert, säurefrei gewaschen und bei 70 bis 80°C getrocknet.

Ausbeute an Bis-(2,4-dichlor-5-methylphenyl)-disulfid: 59,4 g (100 % der Theorie);
Reinheitsgrad: 97 %; Fp. 124 bis 126°C.

**Vergleichsbeispiel 15**

Man verfährt nach den Angaben des Beispiels 15, verwendet jedoch 200 ml Wasser statt einer Mischung von 100 ml Wasser und 100 ml Chlorbenzol und setzt keinen Phasentransferkatalysator zu. Nach Zugabe von 1,9 ml Iodwasserstoffsäure 57 %ig und 50 ml Schwefeldioxid zu der wäßrigen Suspension von 77,9 g 2,4-Dichlor-5-methylbenzol-sulfochlorid erhält man nach 5 Stunden bei 95 bis 100°C Druckkonstanz bei 7,5 bar. Man kühlt den Autoklaven ab und entspannt. Das Produkt wird durch Filtration isoliert, säurefrei gewaschen und getrocknet. Ausbeute: 41,6 g eines Produktgemisches mit einem Gehalt an Bis-(2,4-dichlor-5-methylphenyl)-disulfid von ca. 30 %; Ausbeute: ca. 22 % der Theorie; Fp. 70 bis 73°C.

**Beispiel 16 bis 19**

Der nachfolgenden Tabelle II sind die Ergebnisse weiterer Reduktionen von 77,9 g (0,3 mol) 2,4-Dichlor-5-methylphenylsulfochlorid zu Bis-(2,4-dichlor-5-methylphenyl)-disulfid zu entnehmen, die unter den in Beispiel 15 angegebenen Reaktionsbedingungen, jedoch in Gegenwart des angegebenen 2-Phasensystems und der ange-

gebenen Phasentransferkatalysatoren durchgeführt wurden.

**TABELLE II**

| Beispiel Nr. | 2-Phasensystem | Phasentransfer-katalysator | Bis-(2,4-dichlor-5-metylphenyl)-disulfid Ausbeute in % d.Th. | Schmelzpunkt in °C |
|---|---|---|---|---|
| 16 | 100 ml Toluol<br>50 ml Wasser | 1,6 g B | 95 | 123—125 |
| 17 | 100 ml o-Xylol<br>100 ml Wasser | 1,5 g H | 96 | 125—127 |
| 18 | 70 ml o-Dichlorbenzol<br>100 ml Wasser | 1,3 g L | 95 | 124—126 |
| 19 | 100 ml sym. Tetrachlorethan<br>100 ml Wasser | 1,5 g M | 93 | 123—126 |

In der vorstehenden Tabelle bedeuten:
B: vgl. Erläuterung zu Tabelle I
H: Dodecyl-benzyl-dimethyl-ammoniumchlorid
L: Heneicosyl-trimethyl-ammoniumchlorid (Heneicosyl ist dabei eine Mischung von $C_{20}H_{41}$ bis $C_{22}H_{45}$-Alkylresten)
M: Tetradecyl-benzyl-dimethyl-ammoniumchlorid

**Beispiel 20**

In einem 500 ml Tantal-Autoklaven werden 73,7 g (0,3 mol) 2,5-Dichlorbenzol-sulfochlorid in Form eines 82,4 %igen Filterkuchens (Herstellung durch Sulfochlorierung von 1,4-Dichlorbenzol mit Chlorsulfonsäure und anschließende Fällung auf Eis) in eine Mischung von 100 ml Wasser, 100 ml Chlorbenzol, 2,7 g des in Beispiel 1 benutzten Phasentransferkatalysators (50 %ig in Chlorbenzol) und 2 ml Iodwasserstoffsäure 57%ig eingetragen. Man drückt 50 ml Schwefeldioxid auf, heizt auf 100°C und rührt 5 Stunden bei dieser Temperatur bis zur Druckkonstanz von 5,4 bar nach. Das Chlorbenzol wird darauf azeotrop abdestilliert. Das Produkt wird bei ca. 30°C durch Filtration isoliert, säurefrei gewaschen und bei 60°C getrocknet.

Ausbeute an Bis-(2,5-dichlorphenyl)-disulfid 52,8 g (98 % der Theorie); Reinheitsgrad: 99 %; Fp. 79 bis 81°C.

**Vergleichsbeispiel 20**

73,7 g (0,3 mol) 2,5-Dichlorbenzol-sulfochlorid, technisch feucht, 82,4 %ig, werden in 200 ml Wasser eingetragen und mit 2 ml Iodwasserstoffsäure versetzt. Dieses Gemisch wird in einem 500 ml Tantal-Autoklaven mit 40 ml Schwefeldioxid versetzt und auf 100°C geheizt. Nach 5 Stunden stellt sich bei dieser Temperatur ein konstanter Druck von 8 bar ein. Man kühlt auf 20°C und entspannt den Autoklaven. Man erhält 55 g eines braunen, wasserunlöslichen Öls, in dem sich chromatographisch ca. 25 % Bis-(2,5-dichlorphenyl)disulfid nachweisen läßt (Ausbeute: ca. 26 % der Theorie).

**Beispiel 21**

71,6 g (0,3 mol) 4-Chlor-2,5-dimethylbenzol-sulfochlorid werden in 60 %iger, wasserfeuchter Form (Herstellung durch Sulfochlorierung von 2-Chlor-1,4-dimethylbenzol mit Chlorsulfonsäure und Isolierung durch Fällung auf Eiswasser) bei 20°C in eine Mischung von 100 ml Wasser, 100 ml Chlorbenzol, 2 ml Iodwasserstoffsäure 57 %ig und 3,9 g des in Beispiel 1 verwendeten Phasentransferkatalysators (50 %ig in Chlorbenzol) eingetragen. Das Gemisch wird in einem 500 ml Email-Autoklaven mit 50 ml Schwefeldioxid versetzt, langsam auf 100°C geheizt und 7 Stunden bei 100°C bis zur Druckkonstanz von 7 bar gerührt. Anschließend wird auf 20 bis 25°C gekühlt, entspannt, das organische Lösungsmittel durch Wasserdampfdestillation regeneriert und kaltgerührt. Das Produkt wird filtriert, säurefreigewaschen und getrocknet.

Ausbeute: 46,1 g (85 % der Theorie) Bis-(4-chlor-2,5-dimethyl)-disulfid; Reinheitsgrad: 95%; Fp. 100 bis 102°C.

**Vergleichsbeispiel 21**

Eine Mischung von 71,6 g (0,3 mol) 4-Chlor-2,5-dimethylbenzol-sulfochlorid und 2 ml Iodwasserstoffsäure

57 %ig in 200 ml Wasser wird in einem 500 ml Email-Autoklaven mit 50 ml Schwefeldioxid versetzt und langsam auf 100°C geheizt. Man rührt 7 Stunden bei 100°C nach und erreicht Druckkonstanz bei 11,5 bar. Anschließend wird kaltgerührt und entspannt. Man erhält 0,8 g eines schmierigen Produkts (verunreinigtes Bis-(4-Chlor-2,5-dimethylphenyl)-disulfid), das durch Filtration entfernt wird. Das wäßrige Filtrat wird mit 88 ml 10 n Natronlauge auf pH 7 gestellt. Das ausgefallene Produkt wird durch Filtration isoliert und bei 100°C getrocknet.

Ausbeute: 61,8 g 4-Chlor-2,5-dimethyl-benzolsulfinsaures Natrium; Fp.> 300°C.

**Beispiel 22**

97 g (0,1 mol) Kupferphthalocyanin-tetrasulfochlorid werden als 38 %ige, technisch feuchte Paste (Herstellung aus 57,6 g Kupferphthalocyanin, Chlorsulfonsäure und Thionylchlorid bei erhöhter Temperatur) in eine Mischung von 350 ml Chlorbenzol, 100 ml Wasser, 2,6 ml Iodwasserstoffsäure 57 %ig und 3,6 g des in Beispiel 1 benutzten Phasentransferkatalysators (50 %ig in Chlorbenzol) eingetragen. Aus einem Druckgefäß drückt man unter Rühren in einen 1,3 l Tantal-Autoklaven bei 20 bis 25°C 66 ml Schwefeldioxid zu und heizt das Gemisch auf 95 bis 100°C. Es wird 6 Stunden bei dieser Temperatur bis zur Druckkonstanz von 2,6 bar nachgerührt. Darauf wird gekühlt und die Apparatur entspannt. Chlorbenzol wird aus der grünen Reaktionsmischung durch azeotrope Destillation entfernt. Anschließend kühlt man auf ca. 50°C, filtriert und wäscht den in Pigmentform anfallenden, wasserunlöslichen grünen Schwefelfarbstoff säurefrei.

Ausbeute: 318 g grüne Farbstoffpaste; nach dem Trocknen: 80 g grünes Pulver.

In der Leukoform weist der erhaltene Schwefelfarbstoff eine hohe Löslichkeit auf und ergibt auf Baumwolle brillante grüne Färbungen hoher Farbstärke mit sehr guten Echtheitseigenschaften.

**Beispiel 23**

Die Herstellung einer Lösung von 8-Chlornaphthalin-1-sulfochlorid aus 79,4 g (0,3 mol) 8-Chlornaphthalin-2-sulfonsaurem Natrium wird entsprechend den Angaben des Beispiels 1 durchgeführt.

Man legt 300 ml Wasser vor und dosiert die Lösung von 8-Chlornaphthalin-1-sulfochlorid in Chlorbenzol langsam zu. Anschließend werden 1,9 ml Iodwasserstoffsäure und 2,5 g einer 50 %igen Chlorbenzollösung des in Beispiel 1 benutzten Phasentransferkatalysators zugegeben. Darauf versetzt man die Mischung bei 20 bis 25°C mit 121 g einer 50 %igen wäßrigen Lösung von hypophosphoriger Säure und rührt das Gemisch 4 Stunden bei 80 bis 85°C. Chlorbenzol wird darauf durch azeotrope Destillation entfernt. Man kühlt auf 30°C, filtriert, wäscht das Produkt säurefrei und trocknet bei 80°C.

Ausbeute: 59,9 g (99 % der Theorie) Bis-(8-chlornaphthyl-1)-disulfid; Reinheitsgrad: 95%; Fp. 172 bis 174°C.

**Beispiel 24**

Man verfährt nach den Angaben des Beispiels 23, fügt jedoch zu der Mischung von 0,3 mol 8-Chlornaphthalin-1-sulfochlorid und 2,5 g des Phasentransferkatalysators in Chlorbenzol/Wasser zunächst 1,1 g Jod und darauf 40,5 g Natriumhypophosphit-Monohydrat zu und rührt das Reaktionsgemisch 3 Stunden bei 80 bis 85°C nach. Die Aufarbeitung erfolgt nach den Angaben des Beispiels 23.

Ausbeute: 57,5 g (98 % der Theorie) Bis-(8-chlornaphthyl-1)-disulfid; Reinheitsgrad: 98 %ig; Fp. 172 bis 174°C.

**Beispiel 25**

Man verfährt nach den Angaben des Beispiels 23, setzt jedoch zum Gemisch von 0,3 mol 8-Chlornaphthalin-1-sulfochlorid 1,9 ml Jodwasserstoffsäure und 2,5 g des Katalysators in Chlorbenzol/Wasser statt 121 g 50 %iger hypophosphoriger Säure 126 g einer 70 %igen wäßrigen Lösung von phosphoriger Säure zu.

Ausbeute: 59,0 g (100 % der Theorie) Bis-(8-chlornaphthyl-1)-disulfid; Reinheitsgrad: 98,5 %;Fp. 174 bis 175°C.

**Beispiel 26**

Verfährt man nach den Angaben des Beispiels 23, setzt jedoch statt 121 g 50 %iger hypophosphoriger Säure eine Lösung von 43,8 g Dihydrazin-sulfat in 50 ml Wasser ein, so erhält man 58,7 g (100 % der Theorie) Bis-(8-chlornaphthyl-1)-disulfid, das einen Reinheitsgrad von 99 % und einen Schmelzpunkt von 174 bis 175°C aufweist.

**Beispiel 27**

Eine Emulsion von 97 g p-Fluorbenzolsulfochlorid und 250 ml Wasser wird in einem 700 ml Tantal-Autoklaven mit 3,2 g Iod und 2,8 g des Phasentransferkatalysators Dodecyl-benzyl-dimethyl-ammoniumchlorid versetzt. Anschließend drückt man 80 ml Schwefeldioxid zu, heizt langsam auf 100°C, rührt 8 Stunden bis zur Druckkonstanz von 5,5 bar nach und kühlt darauf auf 20 bis 25°C. Nach dem Entspannen wird das als gelbe Flüssigkeit anfallende Bis-(4-fluorphenyl)-disulfid von der wäßrigen Phase abgetrennt.

Ausbeute: 57,2 g (90 % der Theorie) gelbes Öl. $Kp_{2,39\ mbar}$: 115 bis 120°C.

In der Tabelle III sind weitere Sulfochloride aufgeführt, die nach dem erfindungsgemäßen Verfahren in die entsprechenden Disulfide überführt wurden. Im einzelnen ist in Tabelle III angegeben:

das eingesetzte Aryl- bzw. Hetaryl-sulfochlorid oder der sulfochloridgruppenhaltige Farbstoff; die Verfahrensweise analog einem der bereits beschriebenen Beispiele; die Ausbeute in % der Theorie an dem erhaltenen Disulfid und sein Schmelzpunkt in °C.

**TABELLE III**

| Sulfochlorid-Ausgangsprodukt | Verfahren analog Beispiel | Ausbeute in % d. Th. | Disulfid-Endprodukt | Schmelzpunkt (Siedepunkt) (°C) |
|---|---|---|---|---|
| Benzol-sulfochlorid | 15 | 92 | Diphenyl-disulfid | 58 bis 60 |
| p-Toluol-sulfochlorid | 15 | 95 | Bis-(p-tolyl)-disulfid | 43 bis 45 |
| p-Chlorbenzol-sulfochlorid | 15 | 95 | Bis-(p-chlorphenyl)-disulfid | 67 bis 69 |
| Naphtalin-2-sulfochlorid | 15 | 90 | Dinaphtyl(2)-disulfid | 135 bis 137 |
| 4-Acetaminobenzol-sulfochlorid | 15 | 90 | 4,4'-Diaminodiphenyl-disulfid | 74 bis 76 |
| Thiophen-2-sulfochlorid | 15 | 90 | Di-2-thienyl-disulfid | 52 bis 54 |
| Pyridin-3-sulfochlorid | 15 | 92 | Di-3-pyridyl-disulfid | 186 bis 188 (Dipikrat) |
| 2-Cyano-5-ethoxybenzol-sulfochlorid | 1 (Reduktionstemp.: 40 bis 45°C) | 89 | Bis-(2-carbonamido-5-ethoxy-phenyl)-disulfid | 242 bis 246 |
| Benzol-1,3-disulfochlorid | 15 | 90 | polymeres Benzol-1,3-disulfid | kein definierter Schmelzpunkt |
| Kupferphtalocyanin-disulfochlorid | 23 | | blauer Schwefelfarbstoff (Pigmentform) | " |
| Kobaltphthalocyanin-trisulfochlorid | 23 | | blaugrüner Schwefelfarbstoff (Pigmentform) | " |
| Nickelphtalocyanin-tetrasulfochlorid | 23 | | grüner Schwefelfarbstoff (Pigmentform) | " |
| Tetrasulfochlorid von CI Pigment Violet 23 | 23 | | blauer Schwefelfarbstoff (Pigmentform) | " |
| 6,13-Dichlor-triphenodioxazin-disulfochlorid | 23 | | roter Schwefelfarbstoff (Pigmentform) | " |

**Patentansprüche**

1. Verfahren zur Herstellung von aromatischen Disulfiden, heteroaromatischen Disulfiden oder Farbstoff-Disulfiden, mit Ausnahme von Nitrogruppen enthaltenden aromatischen, heteroaromatischen und Farbstoff-disulfiden, durch Reduktion der entsprechenden Sulfochloride mit Iodwasserstoffsäure oder mit Iodwasserstoffsäure liefernden Substanzen in Gegenwart von Reduktionsmitteln, die Iod in Iodwasserstoffsäure überführen, dadurch gekennzeichnet, daß die Reaktion in einem aus Wasser und einem organischen Lösungsmittel, oder in einem aus Wasser und einem als Ausgangsprodukt dienenden flüssigen Sulfochlorid bestehenden 2-Phasensystem in Gegenwart eines Phasentransferkatalysators durchgeführt wird.

2. Verfahren nach Anspruch 1, dadurch gekennzeichnet, daß die Reaktion bei einer Temperatur von 20 bis 200°C durchgeführt wird.

3. Verfahren nach Anspruch 1 oder 2, dadurch gekennzeichnet, daß die Reaktion bei einer Temperatur von 40 bis 150°C durchgeführt wird.

4. Verfahren nach einem oder mehreren der Ansprüche 1 bis 3, dadurch gekennzeichnet, daß die Reaktion in Gegenwart eines zusätzlichen Tensids durchgeführt wird.

5. Verfahren nach einem oder mehreren der Ansprüche 1 bis 4, dadurch gekennzeichnet, daß pro mol Ausgangsverbindung 0,0001 bis 0,05 mol des Phasentransferkatalysators eingesetzt werden.

6. Verfahren nach einem oder mehreren der Ansprüche 1 bis 5, dadurch gekennzeichnet, daß pro mol Ausgangsverbindung 0,001 bis 0,02 mol des Phasentransferkatalysators eingesetzt werden.

7. Verfahren nach einem oder mehreren der Ansprüche 1 bis 6, dadurch gekennzeichnet, daß der Phasentransferkatalysator ein Oniumsalz der Formel

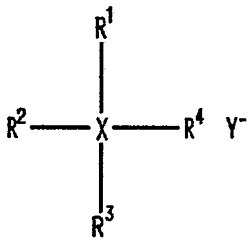

(Ia)

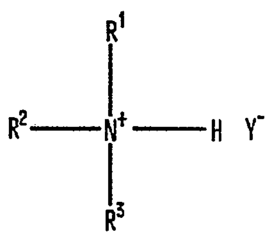

(Ib)

ist, worin $R^1$, $R^2$, $R^3$, $R^4$ gleiche oder verschiedene Alkylreste mit 1 bis 25 C-Atomen, Hydroxyalkylreste mit 2 bis 25 C-Atomen, Arylreste, Aralkylreste und/oder Cycloalkenylreste und $X^+ = N^+$, $P^+$, $As^+$ oder $S^+$ und $Y^-$ ein Anion bedeuten und zwei der Reste $R^1$, $R^2$, $R^3$, $R^4$ zusammen mit dem $X^+$ oder $N^+$ auch ein Ringsystem mit 5 bis 7 C-Atomen bilden können.

8. Verfahren nach einem oder mehreren der Ansprüche 1 bis 7, dadurch gekennzeichnet, daß der Phasentransferkatalysator ein makrocyclischer Polyether der Formel

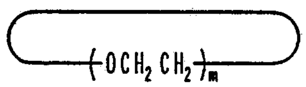

(II)

ist, worin m 4 bis 12 bedeutet und ein, mehrere oder alle Sauerstoffatome auch durch -NH-, -S-, -CO-, -CS- und/oder phosphorhaltige Gruppen und/oder ein, mehrere oder alle Ethanogruppen ($-CH_2OCH_2-$) auch durch Furan-2,5-diyl, Pyridin-2,6-diyl und/oder Thien-2,5-diyl ersetzt sein können und an den Ring auch eine oder mehrere Benzol- und/oder Cyclohexan-Kerne ankondensiert sein können.

9. Verfahren nach einem oder mehreren der Ansprüche 1 bis 8, dadurch gekennzeichnet, daß der Phasentransferkatalysator eine Verbindung der Formeln

$$R^5-(OCH_2CH_2)_p-O-R^6 \quad (III)$$

$$R^5-O-(CH_2CH_2O)_p-(CH_2)_q-CH_2O-R^6 \quad (IV)$$

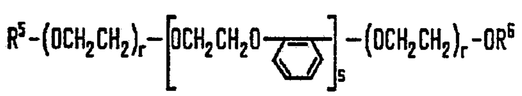

(V)

ist, worin $R^5$, $R^6$ verschieden oder gleich sind und Alkylreste mit 1 bis 15 C-Atomen Chinolyl, Alkylcarbonyl mit 1 bis 15 C-Atomen im Alkylrest, Phenyl, substituiertes Phenyl und p eine ganze Zahl von 3 bis 50, q eine ganze Zahl von 1 bis 10, r eine ganze Zahl von 2 bis 12 und s eine ganze Zahl von 1 bis 6 bedeuten.

10. Verfahren nach einem oder mehreren der Ansprüche 1 bis 9, dadurch gekennzeichnet, daß der Phasentransferkatalysator ein tertiäres Amin der Formel VI

$$N-\left[CHR^7-CHR^8-O-(CHR^9-CHR^{10}-O)_t-R^{11}\right]_3 \quad (VI)$$

ist, worin t eine ganze Zahl von 0 bis 10, $R^7$, $R^8$, $R^9$ und $R^{10}$ gleich oder verschieden sind und ein Wasserstoffatom oder einen Alkylrest mit 1 bis 4 C-Atomen darstellen und $R^{11}$ einen Alkyl- oder Cycloalkylrest mit 1 bis 12

C-Atomen, einen Phenylrest oder einen $C_vH_{2v+1}$-$C_6H_4$- oder -$C_vH_{2v}$-$C_6H_5$-Rest bedeuten, in dem v eine ganze Zahl von 1 bis 12 ist.

## Claims

1. Process for the preparation of aromatic disulphides, hetero-aromatic disulphides or dyestuff disulphides, with the exception of aromatic, hetero-aromatic and dyestuff disulphides containing nitro groups, by reducing the corresponding sulphochlorides with hydriodic acid or with substances which provide hydriodic acid, in the presence of reducing agents which convert iodine into hydriodic acid, characterised in that the reaction is carried out in a 2-phase system consisting of water and an organic solvent or of water and a liquid sulphochloride serving as starting product in the presence of a phase transfer catalyst.

2. Process according to Claim 1, characterised in that the reaction is carried out at a temperature of 20 to 200°C.

3. Process according to Claim 1 or 2, characterised in that the reaction is carried out at a temperature of 40 to 150°C.

4. Process according to one or more of Claims 1 to 3, characterised in that the reaction is carried out in the presence of a surfactant in addition.

5. Process according to one or more of Claims 1 to 4, characterised in that 0.0001 to 0.05 mol of the phase transfer catalyst is employed per mol of starting compound.

6. Process according to one or more of Claims 1 to 5, characterised in that 0.001 to 0.02 mol of the phase transfer catalyst is employed per mol of starting compound.

7. Process according to one or more of Claims 1 to 6, characterised in that the phase transfer catalyst is an onium salt of the formula

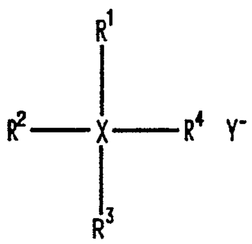
(Ia)

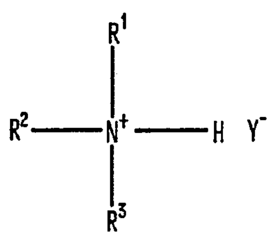
(Ib)

wherein $R^1$, $R^2$, $R^3$ and $R^4$ denote identical or different alkyl radicals having 1 to 25 C atoms, hydroxyalkyl radicals having 2 to 25 C atoms, aryl radicals, aralkyl radicals and/or cycloalkenyl radicals and $X^+$ denotes $N^+$, $P^+$, $As^+$ or $S^+$ and $Y^-$ denotes an anion and two of the radicals $R^1$, $R^2$, $R^3$ and $R^4$, together with $X^+$ or $N^+$, can also form a ring system having 5 to 7 C atoms.

8. Process according to one or more of Claims 1 to 7, characterised in that the phase transfer catalyst is a macrocyclic polyether of the formula

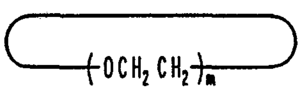
(II)

wherein m denotes 4 to 12 and one oxygen atom, several oxygen atoms or all the oxygen atoms can also be replaced by -NH-, -S-, -CO-, -CS- and/or groups containing phosphorus, and/or one or several or all of the ethano groups (-$CH_2OCH_2$-) can also be replaced by furan-2,5-diyl, pyridine-2,6-diyl and/or thiene-2,5-diyl, and one or more benzene and/or cyclohexane nuclei can also be fused to the ring.

9. Process according to one or more of Claims 1 to 8, characterised in that the phase transfer catalyst is a compound of the formulae

$R^5-(OCH_2CH_2)_p-O-R^6$ (III)

$R^5-O-(CH_2CH_2O)_p-(CH_2)_q-CH_2O-R^6$ (IV)

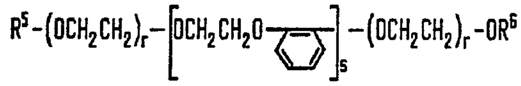 (V)

wherein $R^5$ and $R^6$ are indentical or different and denote alkyl radicals having 1 to 15 C atoms, quinolyl, alkylcarbonyl having 1 to 15 C atoms in the alkyl radical, phenyl or substituted phenyl and p denotes an integer from 3 to 50, q denotes an integer from 1 to 10, r denotes an integer from 2 to 12 and s denotes an integer from 1 to 6.

10. Process according to one or more of Claims 1 to 9, characterised in that the phase transfer catalyst is a tertiary amine of the formula VI

$N-\left[CHR^7-CHR^8-O-(CHR^9-CHR^{10}-O)_t-R^{11}\right]_3$ (VI)

wherein t is an integer from 0 to 10 and $R^7$, $R^8$, $R^9$ and $R^{10}$ are identical or different and represent a hydrogen atom or an alkyl radical having 1 to 4 C atoms and $R^1$ denotes an alkyl or cycloalkyl radical having 1 to 12 C atoms, a phenyl radical or a $C_vH_{2v+1}-C_6H_4-$ or $-C_vH_{2v}-C_6H_5$ radical in which v is an integer from 1 to 12.

**Revendications**

1. Procédé de préparation de disulfures aromatiques, de disulfures hétéro-aromatiques ou de colorants disulfures à l'exception des composés de ce genre qui portent un radical nitro, par réduction des sulfochlorures correspondants au moyen de l'acide iodhydrique ou de substances fournissent de l'acide iodhydrique, en présence de réducteurs transformant l'iode en iodure d'hydrogène, procédé caractérisé en ce qu'on effectue la réaction dans un système à deux phases constitué d'eau et d'un solvant organique, ou constitué d'eau et d'un sulfochlorure liquide servant de corps de départ, en présence d'un catalyseur de transfert de phase.

2. Procédé selon la revendication 1, caractérisé en ce qu'on effectue la réaction à une température de 20 à 200°C.

3. Procédé selon l'une des revendications 1 et 2, caractérisé en ce qu'on effectue la réaction à une température de 40 à 150°C.

4. Procédé selon une ou plusieurs des revendications 1 à 3, caractérisé en ce qu'on effectue la réaction en présence d'un surfactif supplémentaire.

5. Procédé selon une ou plusieurs des revendications 1 à 4, caractérisé en ce qu'on met en jeu, par mole du corps de départ, de 0,0001 à 0,05 mol du catalyseur de transfert de phase.

6. Procédé selon une ou plusieurs des revendications 1 à 5, caractérisé en ce qu'on met en jeu, par mole du corps de départ, de 0,001 à 0,02 mol du catalyseur de transfert de phase.

7. Procédé selon une ou plusieurs des revendications 1 à 6, caractérisé en ce que le catalyseur de transfert de phase est un sel d'onium répondant à l'une des formules :

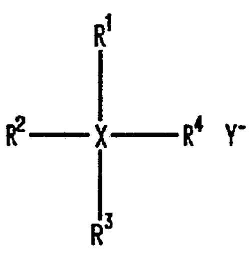

(Ia)

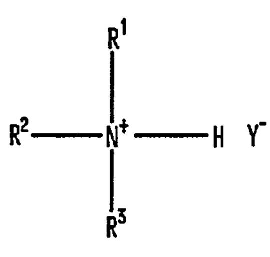

(Ib)

dans lesquelles $R^1$, $R^2$, $R^3$ et $R^4$ représentent chacun, indépendamment les uns des autres, un radical alkyle contenant de 1 à 25 atomes de carbone, un radical hydroxyalkyle contenant de 2 à 25 atomes de carbone, un radical aryle un radical aralkyle ou un radical cyclo-alcényle, $X^+$ représente $N^+$, $P^+$, $As^+$ ou $S^+$, et $Y^-$ représente un anion, deux des radicaux $R^1$, $R^2$, $R^3$ et $R^4$ pouvant aussi former ensemble, avec $X^+$ ou $N^+$, un système cyclique contenant de 5 à 7 atomes de carbone.

8. Procédé selon une ou plusieurs des revendications 1 à 7, caractérisé en ce que le catalyseur de transfert de phase est un polyéther macrocyclique répondant à la formule:

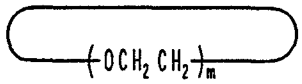

(II)

dans laquelle m représente un nombre de 4 à 12 et un, plusieurs ou tous les atomes d'oxygène peuvent être remplacés par des radicaux -NH-, -S-, -CO-, -CS- et/ou des radicaux phosphorés et/ou un, plusieurs ou tous les radicaux éthano ($-CH_2OCH_2-$) peuvent être remplacés par un radical furanne-diyle-2,5, pyridine-diyle-2,6 et/ou thiophène-diyle-2,5, polyéther dans lequel un ou plusieurs noyaux de benzène et/ou de cyclohexane peuvent être condensés au cycle.

9. Procédé selon une ou plusieurs des revendications 1 à 8, caractérisé en ce que le catalyseur de transfert de phase est un composé répondant à l'une des formules :

$R^5-(OCH_2CH_2)_p-O-R^6$ (III)

$R^5-O-(CH_2CH_2O)_p-(CH_2)_q-CH_2O-R^6$ (IV)

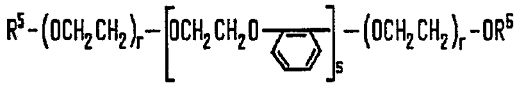

(V)

dans lesquelles $R^5$ et $R^6$ représentent chacun, indépendamment l'un de l'autre, un radical alkyle contenant de 1 à 15 atomes de carbone, un radical quinoléyle, un radical alkylcarbonyle dont la partie alkyle contient de 1 à 15 atomes de carbone, un radical phényle ou un radical phényle substitué, p représente un nombre entier de 3 à 50, q un nombre entier de 1 à 10, r un nombre entier de 2 à 12 et s un nombre entier de 1 à 6.

10. Procédé selon une ou plusieurs des revendications 1 à 9, caractérisé en ce que le catalyseur de transfert de phase est une amine tertiaire répondant à la formule VI :

$N-[CHR^7-CHR^8-O-(CHR^9-CHR^{10}-O)_t-R^{11}]_3$ (VI)

dans laquelle t représente un nombre entier de 0 à 10, $R^7$, $R^8$, $R^9$ et $R^{10}$ représentent chacun, indépendamment les uns des autres, un atome d'hydrogène ou un radical alkyle contenant de 1 à 4 atomes de carbone, et $R^{11}$ représente un radical cycloalkyle ou alkyle contenant de 1 à 12 atomes de carbone, un radical phényle ou un radical $-C_vH_{2v+1}-C_6H_5$ ou $-C_vH_{2v}-C_6H_5$ dans lequel v représente un nombre entier de 1 à 12.